(19) **Europäisches Patentamt**

**European Patent Office**

**Office européen des brevets**

(11) **EP 0 789 031 A2**

(12) **EUROPEAN PATENT APPLICATION**

(43) Date of publication:
**13.08.1997 Bulletin 1997/33**

(51) Int. Cl.$^6$: **C07K 14/235**, A61K 39/10

(21) Application number: **97104861.6**

(22) Date of filing: **23.12.1991**

| | |
|---|---|
| (84) Designated Contracting States:<br>**AT BE CH DE ES FR GB IT LI NL**<br><br>(30) Priority: **21.12.1990 GB 9027901**<br><br>(62) Document number(s) of the earlier application(s) in accordance with Art. 76 EPC:<br>**92901932.1 / 0 573 435**<br><br>(71) Applicant: **EVANS MEDICAL LIMITED Leatherhead, Surrey KT22 7PQ (GB)**<br><br>(72) Inventors:<br>  • **Charles, Ian George**<br>    **London W1P 9LN (GB)** |   • **Dougan, Gordon,**<br>    **Dept. of Biochemistry**<br>    **London SW7 2AZ (GB)**<br>  • **Li, Jing Li,**<br>    **Dept. of Biochemistry**<br>    **Exhibition Road, London SW7 2AZ (GB)**<br><br>(74) Representative: **Woods, Geoffrey Corlett**<br>    **J.A. KEMP & CO.**<br>    **14 South Square**<br>    **Gray's Inn**<br>    **London WC1R 5LX (GB)**<br><br><u>Remarks:</u><br>    This application was filed on 21 - 03 - 1997 as a divisional application to the application mentioned under INID code 62. |

(54) **Acellular vaccine**

(57) An antigenic fragment of a *Bordetella parapertussis* antigen, the said antigen having an amino acid sequence as set out in SEQ ID NOS: 1 and 2 or an amino acid sequence that is at least 94% homologous with that set out in SEQ ID NOS: 1 and 2.

## Description

The invention relates to the isolation and characterisation of an antigen of <u>Bordetella</u> <u>parapertussis</u> and the antigenic sites, DNA sequences encoding the antigen, expression vector& containing such DNA sequences, host cells transformed by such expression vectors. The invention also relates to the use of the antigen in a vaccine for the prevention of <u>B.parapertussis</u> infections.

<u>B.pertussis</u> causes a serious and debilitating disease in humans, especially children. It has been kept under control in the developed countries by large scale immunisation programmes. Traditionally, immunisation has been carried out using a whole cell vaccine derived from cell cultures of <u>B.pertussis</u> and has been found to be relatively effective in preventing the disease.

Concern over adverse reactions, such as fever, local reactions and persistent screaming, has led in recent years to a reduced acceptance of the whole cell <u>B.pertussis</u> vaccine and debate about its continued use. Research has therefore been directed towards the development of vaccines which lack the components responsible for the adverse effects of the whole cell vaccines, whilst retaining the components necessary to confer protection against the disease.

The search for safe and effective vaccines has been hampered by the paucity of information regarding the identity and mechanisms of action of the pathogenic, toxic and protective moieties of the bacterial organism contained in whole cell vaccines. The search for effective vaccines has, therefore, focused on the isolation and characterisation of surface antigens of the organism and on their efficacy in inducing an immune reaction (<u>J.Am.Med.Soc.</u>, 1982, <u>248</u>(1) 22-23). Examples of <u>B.pertussis</u> antigens that have been investigated include lymphocytosis promoting factor (LPF, otherwise known as pertussis toxin (PT)), filamentous haemagglutinin (FHA), lipopolysaccharide (LPS), agglutinogens, dermonecrotic toxin (DNT), heat labile and heat stable toxins, polymorphonuclear leukocyte inhibitor factor, adenylate cyclase, the outer membrane 69kDa antigen (P.69, pertactin) and other surface components.

<u>B.parapertussis</u> is closely related to <u>B.pertussis</u> and is also responsible for outbreaks of whooping cough in man (Zeulzer <u>et</u> <u>al</u>, J. Pediatr. 9:493-497 (1946); Linneman <u>et</u> <u>al</u> J. Pediatr. 19: 229-240 (1977); Novotny, J.Infect. Dis. 161:581-582, 1990)). However, to date no vaccine specific for <u>B.parapertussis</u> has been developed.

<u>B.parapertussis</u> is also known to produce a number of virulence factors (Pitmann, Bergey's Manual of Systematic Bacteriology Vol I P388-393 Ed. Kreig & Hoft 1984) including FHA, and adenylate cyclase (AdeCase) toxin. Because of the immunological cross-reactivity of these virulence factors, vaccination against whooping cough with a whole cell vaccine may provide some protection against <u>B.parapertussis</u>. However, <u>B.parapertussis</u> does not produce the well characterised pertussis toxin (PT). Thus, outbreaks of <u>B.parapertussis</u> mediated whooping cough are unlikely to be prevented by acellular vaccines composed primarily of detoxified pertussis toxin (PT).

Furthermore, if vaccination programmes against <u>B.pertussis</u> are successful in reducing the incidence of the bacterial organism in the human population, or even in eradicating the organism altogether, just as smallpox has been eradicated by rigorous vaccination programmes, there is a danger that <u>B.parapertussis</u> infections will increase in number and <u>B.parapertussis</u> will become the primary cause of whooping cough. It is therefore desirable to include in any whooping cough vaccination programme, a <u>B.parapertussis</u> specific vaccine, preferably an acellular vaccine.

I.G. Charles <u>et</u> <u>al</u> Proc. Natl. Acad Sci USA Vol 80 pp 3554-3558 (1989) purport to identify homologous proteins in <u>B.pertussis</u> and <u>B.parapertussis</u> with molecular masses of 69 and 70kDa respectively. The assertion of homology is based on the observation that these antigens bind to the BB05 antibody raised against <u>B.bronchiseptica</u>. These proteins although clearly related to each other in their ability to bind BB05 antibody have different immunogenic properties. It is not stated whether the antigen of <u>B.parapertussis</u> would be expected to have any immunogenic potential <u>in</u> <u>vivo</u>, this being necessary for use as an effective vaccine.

The inventors have identified, isolated and characterised an antigen of <u>B.parapertussis</u> described hereinafter, which is useful as the basis for an accelullar vaccine specific for <u>B.parapertussis</u> or as part of an acellular whooping cough vaccine.

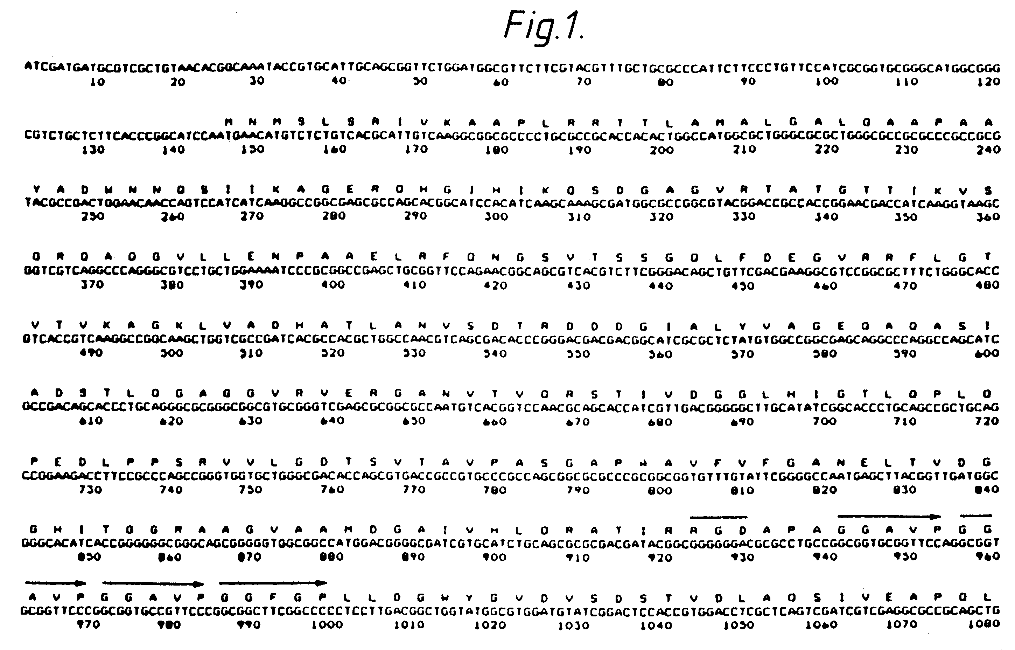

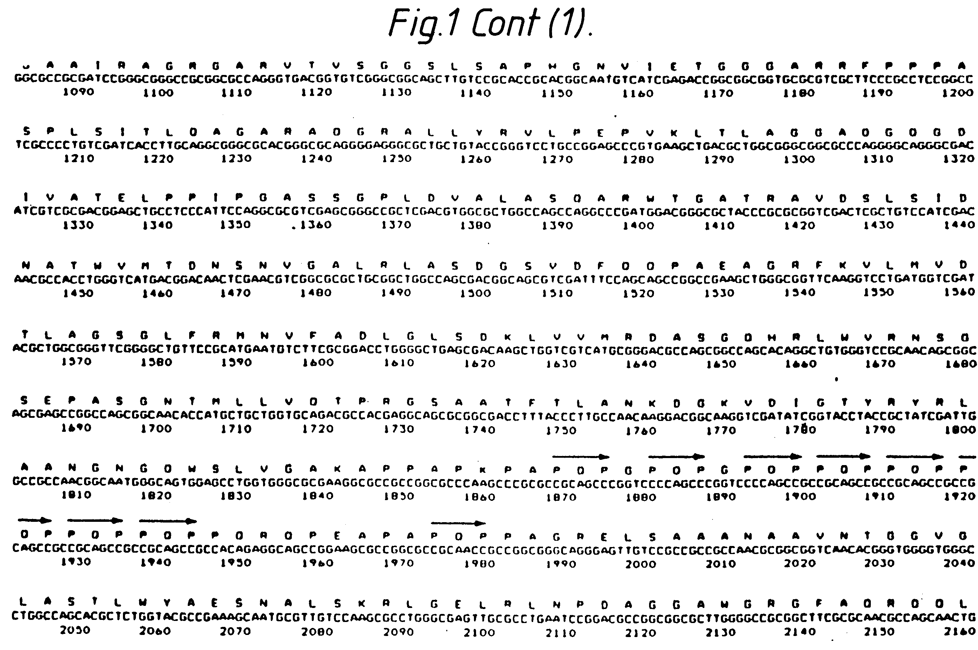

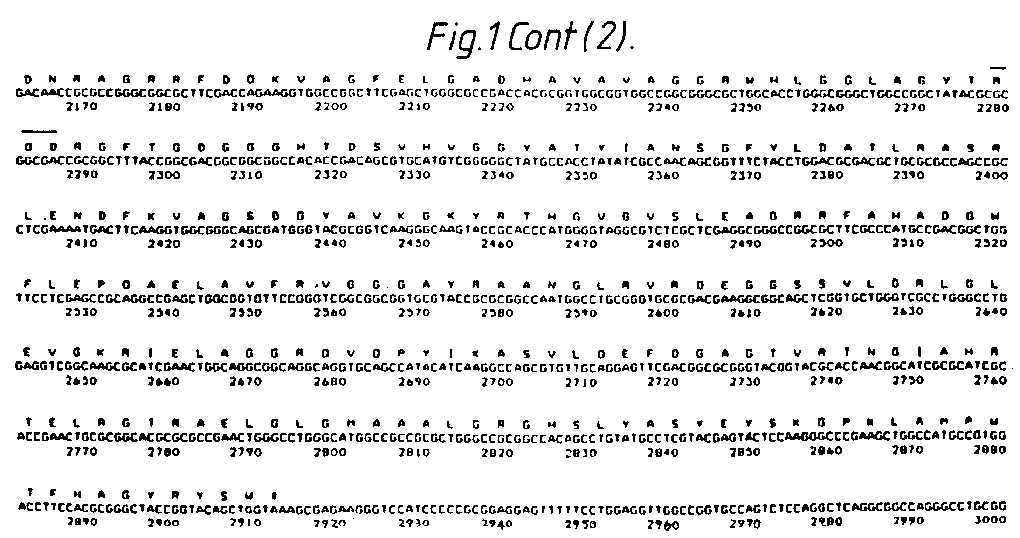

Accordingly, the invention provides a <u>B.parapertussis</u> antigen comprising an amino acid sequence as set out in Figure 1, or an amino acid sequence that is at least 94% homologous with that set out in Figure 1, or an antigenic fragment thereof.

The invention also provides a protein which is uncontaminated by components from <u>B.parapertussis</u>, which is capable of binding to antibody which also binds the native P70 antigen of <u>B.parapertussis</u> and which has the amino acid sequence as set out in Figure 1 from amino acid residue Asp 35-Asn 643, or an amino acid sequence that is at least 94% homologous with that set out in Figure 1 from amino acid residue Asp 35 - Asn 643, or an antigenic fragment thereof.

The invention also includes the amino acid sequence set out in Figure 1, or one which is at least 95% or even 98% homologous with that set out in Figure 1.

The amino acid sequence of Figure 1 shows a precursor protein of molecular weight approximately 95,000 Daltons (P.95) comprising 922 amino acids. <u>In</u> <u>vivo</u> processing of this precursor yields a protein with an estimated molecular weight of 70,000 Daltons (P.70) as determined by SDS polyacrylamide gel electrophoresis, but the actual weight is approximately 61 kDa.

The invention therefore provides as a preferred feature, the amino acid sequence of Figure 1 comprising the amino acid sequence from Asp-35 to Asn 643 or an antigenic fragment thereof.

An antigenic fragment of the amino acid sequence set forth in Figure 1 or of a sequence which is at least 94% homologous therewith preferably includes amino acids Pro577 to Pro 612 or Ala 574 to Pro 612.

The antigenic sites of the amino acid sequence set out in Figure 1 may be identified using standard procedures. These may involve fragmentation of the polypeptide itself using proteolytic enzymes or chemical agents and then determining the ability of each fragment to bind to antibodies or to provide an immune response when innoculated into an animal or suitable in vitro model system (Strohmauer et al, J.Gen.Virol, 1982, 59, 205-306). Alternatively, the DNA encoding the polypeptide may be fragmented by restriction enzyme digestion or other well-known techniques and then introduced into an expression system to produce fragments (optionally fused to a polypeptide usually of bacterial origin). The resulting fragments are assessed as described previously (Spence et al, J.Gen.Virol., 1989, 70, 2843-51; Smith et al, Gene, 1984, 29, 263-9). Another approach is to chemically synthesise short peptide fragments (3-20 amino acids long; conventionally 6 amino acids long) which cover the entire sequence of the full-length polypeptide with each peptide overlapping the adjacent peptide. (This overlap can be from 1-10 amino acids but ideally is n-1 amino acids where n is the length of the peptide; Geysen et al, Proc. Natl. Acid. Sci., 1984, 81, 3998-4002). Each peptide is then assessed as described previously except that the peptide is usually first coupled to some carrier molecule to facilitate the induction of an immune response. Finally, there are predictive methods which involve analysis of the sequence for particular features, e.g. hydrophilicity, thought to be associated with immunologically important sites (Hopp and Woods, Proc. Natl. Acad. Sci., 1981, 78, 3824-8; Berzofsky, Science, 1985, 229, 932-40). These predictions may then be tested using the recombinant polypeptide or peptide approaches described previously.

The antigen of the present invention is located on the surface of B.parapertussis, and may be isolated from cultures of B.parapertussis by conventional methods, for example as described by Novotny et al (1985) Infection and Immunity 50, 199-206 and European Publication No. 0162639. Alternatively, it may be obtained using recombinant DNA technology. In this regard, the DNA encoding the antigen may be cloned, for example as described by Makoff et al (Bio-Technology November 1990), inserted into an expression vector which is used to enable expression in an appropriate host.

The invention therefore provides a cloned DNA sequence encoding the antigen of the present invention, preferably the DNA sequence is as set out in Figure 1 or is at least 92% homologous with that set out in Figure 1. The invention also includes a DNA sequence encoding the precursor protein namely from nucleotide 145 to nucleotide 2910 or a DNA sequence encoding the P70 protein namely from nucleotide 247 to nucleotide 2073.

The antigen is preferably provided in a pure form ie. greater than 90% most preferably greater than 95% pure but at least to a level consistent with its use in a human vaccine.

The cloning of the DNA sequence may be carried out using standard procedures known in the art. However, it is particularly advantageous in such procedures to employ the sequence data disclosed herein so as to facilitate the identification and isolation of the desired cloned DNA sequences. Preferably, the DNA is isolated by the method described by Hull et al Infect. Immun. 33: 933-938 (1981) as modified by Maskell et al J.Bacteriol. 170: 2467-2471 (1988). The DNA is then digested with a restriction enzyme to generate short fragments which are then inserted into a cloning vector, such as the cosmid pHC79 or a derivative thereof and the resulting recombinant DNA molecules used to transform E.coli and thus generate the desired library.

The library may be screened using a standard screening strategy. For example one may employ as hybridisation probes one or more labelled oligonucleotides synthesised using the DNA sequence information disclosed herein. One or more additional rounds of screening of one kind or another may be carried out to characterise and identify positive clones.

Having identified a first positive clone, the library may be rescreened for additional positive clones using the first clone as an hybridization probe. Alternatively or additionally, further libraries may be prepared and these may be screened using hybridisation probes. In this way, further DNA sequences may be obtained.

Alternatively, the DNA sequence encoding the antigen of the present invention may be synthesised using standard procedures and this may be preferred to cloning the DNA in some circumstances.

Thus cloned or synthesised, the desired DNA sequence may be inserted into an expression vector using known and standard techniques. The expression vector is normally cut using restriction enzymes and the DNA sequence inserted using blunt-end or staggered-end ligation. The cut is usually made at a restriction site in a convenient position in the expression vector such that, once inserted, the DNA sequence is under the control of the elements of DNA that effect its expression.

These elements may vary according to the host but usually include a promoter, ribosome binding site, translational start and stop sites, and a transcriptional termination site. Examples of such vectors include plasmids and cosmids. Expression vectors of the present invention encompass both extrachromosomal vectors and vectors that are integrated into the host cell's chromosome.

The invention therefore provides an expression vector containing a DNA sequence, as herein defined, and being capable in an appropriate host of expressing the DNA sequence to produce the antigen.

The invention also provides a host cell transformed with an expression vector as herein defined.

Examples of host cells of use with the invention include prokaryotic and eukaryotic cells, such as bacterial, yeast, mammalian and insect cells. Particular examples of such cells are E.coli, S.cerevisiae, P.pastoris, Chinese hamster ovary and mouse cells, and Spodoptera frugiperda and Tricoplusia ni. The choice of host cell may depend on a number of factors but, if post-translational modification of the antigen is important, then a prokaryotic host would be preferred.

Transformation of a host cell may be carried out using standard techniques. Some phenotypic marker is usually employed to distinguish between the transformants that have successfully taken up the expression vector and those that have not. Culturing of the transformed host cell and isolation of the antigen may also be carried out using standard techniques.

The invention thus provides a process for preparing the antigen which comprises cloning or synthesising a DNA sequence encoding the antigen, as herein defined, inserting the DNA sequence into an expression vector such that it is capable in an appropriate host of being expressed, transforming a host cell with the expression vector, culturing the transformed host cell, and isolating the antigen.

The antigen obtained in this way may be insoluble and thus may need to be refolded following the use of guanidinium hydrochloride as denaturant in conventional manner and in any event is preferably purified.

The invention therefore provides a vaccine containing an antigen of B.parapertussis as hereinafter described.

The vaccine of the invention may optionally contain additional antigens of B.parapertussis or other bacteria, such as B.pertussis, tetanus and diphtheria.

The vaccine of the invention is normally associated with a pharmaceutically acceptable vehicle which allows the antigen to be administered to the patient. Administration is usually carried out via the oral, intranasal, or preferably parenteral route. In the case of the parenteral route, the vehicle is generally liquid and the antigen is generally dissolved or suspended in it. An example of a liquid vehicle is physiological saline solution.

The vaccine may also contain an adjuvant for stimulating the immune response and thereby enhancing the potency of the antigen. Convenient adjuvants for use in the present invention include, for example, aluminium hydroxide and aluminium phosphate.

Conveniently the vaccine contains a final concentration of antigen in the range of from 0.01 to 5mg/ml, preferably 0.03 to 2mg/ml, most preferably 0.3mg/ml. After formulation the vaccine may be incorporated into a sterile container which is then sealed and stored at a low temperature, for example 4°C, or is freeze-dried.

The invention also provides a method for inducing immunity to whooping cough in humans, comprising the administration to the patient of an effective amount of the vaccine of the present invention.

In order to induce immunity to whooping cough in humans one or more doses of the vaccine are normally administered. Each dose of the vaccine is 0.1 to 2ml preferably 0.2 to 1ml, most preferably 0.5ml.

The invention will now be exemplified further by reference to the accompanying Figures and the Examples.

Figure Legends

Figure 1. The amino acid sequence of P.70 and the DNA sequence of the prn gene encoding the P.70 antigen from B.parapertussis.

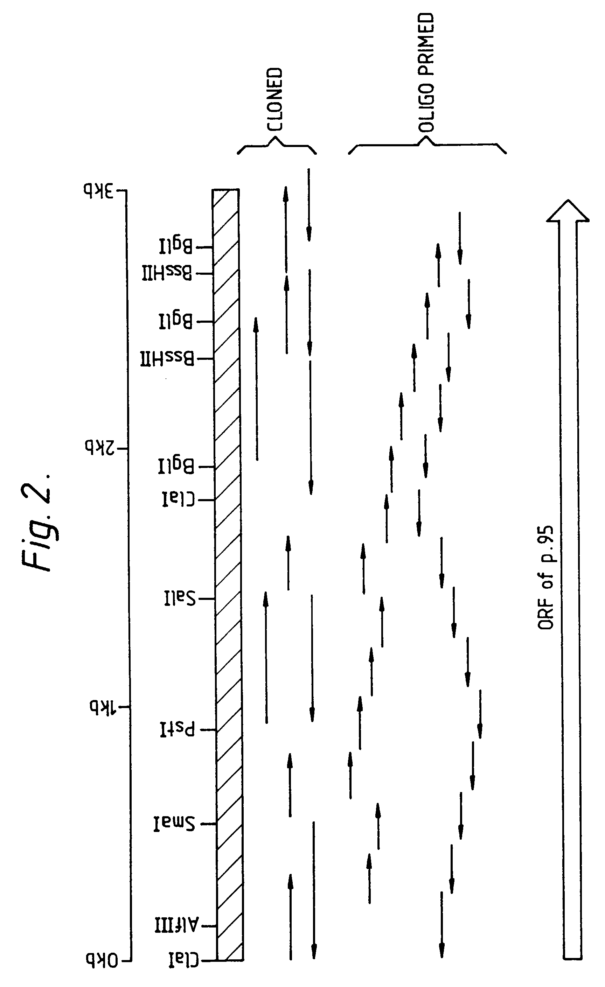

Figure 2. Restriction map and sequencing strategy for the prn gene encoding the P.70 antigen from B.parapertussis. Small arrows represent the direction and extent of DNA sequencing derived from restriction sites or oligonucleotides used as specific primers. The large arrow indicates the direction of transcription of the orf for the gene.

Figure 3. Line drawing showing the strategy used to generate the P.70 antigen expression plasmid pBD845.

Experimental procedures

Bacterial strains, plasmids and phage

B.pertussis strain CN2992, B.parapertussis strain CN2591 were from the Wellcome Culture Collection. E.coli K.12 TGl [Δ (lac-pro) supE, thi, hsdD5/F' traD36 proA$^+$B$^+$, lacl$^q$ lacZ M15 was as described (Carter et al, (1985)] as was E.coli K12 HB101 F$^-$, hsdS20 (r$^-$B m$^-$B) recA13, ara-14, proA2, lacY1, galK2, rpsL20, xyl-5, mtl-1, supE44 (Boyer & Roulland-Dussoix, J.Mol.Biol. 41:459-465, 1969).

Cosmid pHC79 (Hohn & Collins, Gene 11:29-298, 1980) was from Amersham International, Little Chalfont, Buckinghamshire; plasmid pKK223-2 (Amman & Brosius, Gene 40:183-90, 1985) and M13mp18 and H13mp19 (Yanisch-Perron et al, Gene 33:103-119, 1985) were supplied by Pharmacia Ltd., Milton Keynes, Buckinghamshire.

Media and reagents

E.coli strains were grown on Luria broth (LB) or on LB solidified with 1.6% (w/v) agar (Miller, Experiments in Mol.Gene. Cold Spring Harbor N.Y., 1972) (Difco Address). Minimal medium (MM) was made as described by Miller (1972) and was solidified with 2% (w/v) Noble agar (Difco). B.pertussis and B.parapertussis were grown in Stainer-Scholte broth at 37°C (Stainer & Scholte, J.Gen.Microbiol 63:211-220 1962), or on Stainer-Scholte plates solidified with 2% (w/v) Noble agar. Deoxynucleotides, ampicillin, tetracyline and dithiothreitol were from Sigma. Restriction endonucleases and T4 DNA ligase were from BRL, Paisley, Scotland.

Cloning of B.parapertussis chromosomal DNA into cosmid pHC79 and identification of the gene encoding P.70

B.parapertussis chromosomal DNA (prepared by the method of Hull et al Infect.Immun. 33:933-938, 1981 as modified by Maskell et al J.Bacteriol. 170:2467-2471, 1988) was partially digested with Sau3A, and fragments in the 40-50kb size range were ligated into the BamHI site (Maniatis et al Molecular Cloning: Cold Spring Harbor N.Y, 1982; of cosmid pHC79 (Hohn & Collins Gene 11:291-298, 1980). Recombinant cosmids in E.Coli HB101 were plated out and transferred to microtiter plates following the method described by Charles et al (J.Gen.Microbiol. 136:353-358, 1990) and transferred to Gene Screen Plus hybridized membranes (Du Pont, Stevenage, Hertfordshire). The cosmids were then screened for the presence of the prn gene encoding P.70 by DNA : DNA hybridisation using a radioactively labelled 1.8kb Cla1 restriction fragment isolated from the related prn gene from B.pertussis. The Cla1 fragment was gel purified (Tautz & Renz, Anal. Biochem. 132:14-19, 1983) following digestion of cosmid pl69 (Charles et al Proc. Natl. Acad-Sci. USA 86:3554-3558, 1989). The fragment was nick translated with a kit supplied by BCL, Mannheim, and[$\alpha^{32}$ P]-ATP (Amersham), and hybridized with the B.parapertussis gene bank filters as previously described (Charles et al, J.Gen.Microbiol. 136: 353-358 (1990)). Three positive colonies were identified and one, harbouring cosmid pBD811 was selected for further analysis.

Subcloning and DNA sequencing

Restriction fragments of cosmid pBD811 were cloned in M13mp18 and M13mp19 (Yanisch-Perron et al, Gene 33:103-119, 1985) and sequenced using universal primer, [$_\alpha^{-35}$S] dATP (deoxyadenosine 5'-[$_\alpha^{35}$ S] thiotriphosphate) dideoxynucleotide triphosphates, and both gradient and wedge gels (Biggin et al, Proc.Natl. Acad.Sci USA 80:3963-3965, 1983; Sanger et al, Proc.Natl. Acad.Sci. USA 71:5463-5467, 1977). To resolve compression artifacts some clones were sequenced with modified T7 DNA polymerase (Tabor & Richardson, Proc.Natl. Acad.Sci USA 84:4767-4771, 1987) and 7-deaza-2'-dGTP (Misusawa et al, Nucl.Acids.Res. 14:1319-1324, 1986) using a kit supplied by Pharmacia. Gaps in the sequence were filled in using synthetic oligonucleotides as specific primers (Charles et al, Nucl.Acids.Res. 14:2201-2213, 1986).

Oligonucleotides for use as specific sequencing primers were made on a SAM1 oligonucleotide synthesizer (Biolabs).

Computer analysis of the DNA sequence revealed an open reading frame capable of encoding a protein of 922 amino acids with a calculated molecular weight of 95,177.

Expression of P.70 antigen from B.parapertussis on the Surface of E.coli

Using Western blotting, it was not possible to detect the expression of P.70 in E.coli HB101 harbouring cosmid pBD811, although from DNA sequence analysis the cosmid was known to contain the entire structural gene. In order to express the B.parapertussis prn gene in E.coli, the expression vector pKK233-2 (Amann & Brosius, Gene 40:183-90, 1985) was used, which directs high level transcription from a trc promoter.

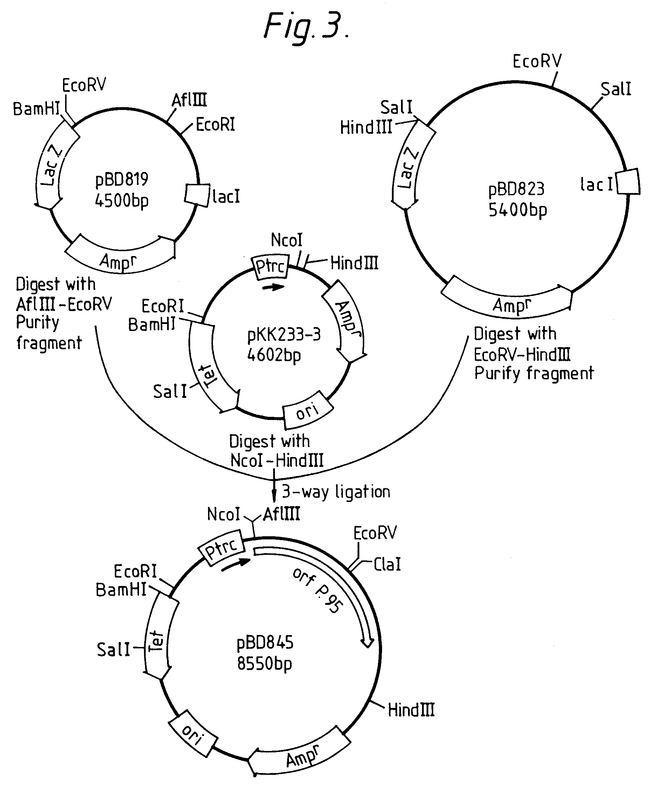

A three-way ligation using Ncol-HindIII digested pKK233-2; a 1.6kb AflIII-EcoRV fragment from the 5' end of P.70 prn and a 2.2 kb EcoRV-HindIII fragment containing the 3' end of P.70 prn was carried out (see Fig. 3). The ligation mix was used to transform E.coli TGI and transformants expressing P.70 antigen identified by their ability to cross-react with the mAb BB05 (Novotny et al, Develop.Biol.Stand. 61:27-41, 1985) in protein dot blots. Colonies returning a positive signal were then isolated, and miniplasmid preps (Maniatis et al, Molecular Cloning: A labaratory Manual, Cold Spring Harbor, N.Y., 1982) carried out to verify that a full-length insert had been cloned. One of the colonies, harbouring plasmid pBD845, was selected for further study. SDS-PAGE electrophoresis was followed by Western blotting. Samples were transferred to nitrocellulose by the method of Towbin (Proc.Natl.Acad-Sci USA 76:4350-4354, 1984). The detection system used was horse radish peroxidase conjugated goat anti-mouse IgG using 4-chloro-1-napthol as substrate (Fairweather et al, J.Bacteriol. 165:21-27, 1986). Western blotting of E.coli TGI harbouring pBD845, (containing the entire structural gene for P.95), shows that a protein with a molecular weight of 95kDa (P.95) is weakly expressed in E.coli, along with a more significant band of 70kDa (P.70). A large number of lower molecular weight species that cross-react with BB05 are also seen suggesting that P.70/P.95 is unstable in E.coli. These observations suggest that the P.95 form of the protein is initially expressed and is subsequently processed to the P.70 form.

Slide agglutination assays. Samples of E.coli strains harbouring pBD845 to be tested ($10^6$-$10^7$) were mixed with 30$\mu$l of IgG purified polyclonal rabbit anti-P.69 antibody on a glass microscope slide and visually scored for clumping after 2 mins as compared with a negative control of either vir⁻ B.parapertussis or E.coli TGI. E.coli strains harbouring pBD845 can be agglutined, and this agglutination occurs just as rapidly as with vir⁺ B.parapertussis suggesting that recombinant P.70 protein is correctly expressed on the surface of E coli.


## Claims

1. An antigenic fragment of a *Bordetella parapertussis* antigen, the said antigen having an amino acid sequence as set out in SEQ ID NOS: 1 and 2 or an amino acid sequence that is at least 94% homologous with that set out in SEQ ID NOS: 1 and 2.

2. A fragment according to claim 1, which is an antigenic fragment of the amino acid sequence of SEQ ID NOS: 1 and 2 from Asp-45 to Asn-643.

3. A fragment according to claim 1, which includes amino acids Pro-577 to Pro-612 or Ala-574 to Pro-612 of the amino acid sequence of SEQ ID NOS: 1 and 2 or of the said sequence at least 94% homologous therewith.

4. A DNA sequence which encodes an antigenic fragment as defined in any one of the preceding claims.

5. An expression vector which contains a DNA sequence as defined in claim 4 and which, when provided in a suitable host, is capable of expressing an antigenic fragment as defined in any one of claims 1 to 3.

6. A vector according to claim 5, which is a plasmid.

7. A host transformed with an expression vector as defined in claim 5 or 6.

8. A host according to claim 7, which is a strain of *E.coli*.

9. A process for the preparation of an antigenic fragment as defined in claim 1, which process comprises maintaining a host transformed with an expression vector which contains a DNA sequence as defined in claim 4 and which is capable of expressing the said fragment in the said host, under such conditions that the said fragment is expressed.

10. A pharmaceutical composition comprising a pharmaceutically acceptable carrier or diluent and, as active ingredient, an antigenic fragment as defined in claim 1.

## Fig.1.

```
ATCGATGATGCGTCGCTGTAACACGGCAAATACCGTGCATTGCAGCGGTTCTGGATGGCGTTCTTCGTACGTTTGCTGCGCCCATTCTTCCCTGTTCCATCGCGGTGCGGGCATGGCGGG
     10        20        30        40         50        60        70        80        90       100       110       120


          M  N  M  S  L  S  R  I  V  K  A  A  P  L  R  R  T  T  L  A  M  A  L  G  A  L  G  A  A  P  A  A
CGTCTGCTCTTCACCCGGCATCCAATGAACATGTCTCTGTCACGCATTGTCAAGGCGGCGCCCCTGCGGCCGCACCACACTGGCCATGGCGCTGGGCGCGCTGGGCGCCGCGCCCGCCGCG
    130       140       150       160       170       180       190       200       210       220       230       240


   Y  A  D  M  N  N  N  Q  S  I  I  K  A  G  E  R  Q  H  G  I  H  I  K  Q  S  D  G  A  G  V  R  T  A  T  G  T  T  I  K  V  S
TACGCCGACTGGAACAACCAGTCCATCATCAAGGCCGGGCGAGCGCCAGCACGGCATCCACATCAAGCAAAGCGATGGCGCCGGCGTACGGACCGCCACCGGAACGACCATCAAGGTAAGC
    250       260       270       280       290       300       310       320       330       340       350       360


   Q  R  Q  A  Q  G  V  L  L  E  N  P  A  A  E  L  R  F  Q  N  G  S  V  T  S  S  G  Q  L  F  D  E  G  V  R  R  F  L  G  T
GGTCGTCAGGCCCAGGGCGTCCTGCTGGAAAATCCCGCGGCCGAGCTGCGGTTCCAGAACGGCAGCGTCACGTCTTCGGGACAGCTGTTCGACGAAGGCGTCCGGCGCTTTCTGGGCACC
    370       380       390       400       410       420       430       440       450       460       470       480


   V  T  V  K  A  G  K  L  V  A  D  H  A  T  L  A  N  V  S  D  T  R  D  D  D  G  I  A  L  Y  V  A  G  E  Q  A  Q  A  S  I
GTCACCGTCAAGGCCGGCAAGCTGGTCGCCGATCACGCCACGCTGGCCAACGTCAGCGACACCCGGGACGACGACGGCATCGCGCTCTATGTGGCCGGCGAGCAGGCCCAGGCCAGCATC
    490       500       510       520       530       540       550       560       570       580       590       600


   A  D  S  T  L  Q  G  A  G  G  V  R  V  E  R  G  A  N  V  T  V  Q  R  S  T  I  V  D  G  G  L  H  I  G  T  L  Q  P  L  Q
GCCGACAGCACCCTGCAGGGCGCGGGCGGCGTGCGGGTCGAGCGCGGCGCCAATGTCACGGTCCAACGCAGCACCATCGTTGACGGGGGGCTTGCATATCGGCACCCTGCAGCCGCTGCAG
    610       620       630       640       650       660       670       680       690       700       710       720


   P  E  D  L  P  P  S  R  V  V  L  G  D  T  S  V  T  A  V  P  A  S  G  A  P  H  A  V  F  V  F  G  A  N  E  L  T  V  D  G
CCGGAAGACCTTCCGCCCAGCCGGGTGGTGCTGGGCGACACCAGCGTGACCGCCGTGCCCGCCAGCGGCGCGCCCGCGGCGGTGTTTGTATTCGGGGCCAATGAGCTTACGGTTGATGGC
    730       740       750       760       770       780       790       800       810       820       830       840


   G  H  I  T  G  G  R  A  A  G  V  A  A  M  D  G  A  I  V  H  L  Q  R  A  T  I  R  R  G  D  A  P  A  G  G  A  V  P  G  G
GGGCACATCACCGGGGGGCGGGCAGCGGGGGTGGCGGCCATGGACGGGGCGATCGTGCATCTGCAGCGCGCGCGACGATACGGCGGGGGGGACGCGCCTGCCGGCGGTGCGGTTCCAGGCGGT
    850       860       870       880       890       900       910       920       930       940       950       960


   A  V  P  G  G  A  V  P  G  G  G  F  G  P  L  L  D  G  W  Y  G  V  D  V  S  D  S  T  V  D  L  A  Q  S  I  V  E  A  P  Q  L
GCGGGTTCCCGGCGGTGCCGGTTCCCGGCGGCTTCGGCCCCCCTCCTTGACGGCTGGGTATGGGCGTGGATGTGTATCGGACTCCACCGTGGACCTCGCTCAGTCGATCGTCGAGGCGCCGCAGCTG
    970       980       990      1000      1010      1020      1030      1040      1050      1060      1070      1080
```

EP 0 789 031 A2

# Fig.1 Cont (1).

```
  G  A  A  I  R  A  G  R  G  A  R  V  T  V  S  G  G  S  L  S  A  P  M  G  N  V  I  E  T  G  G  G  A  R  R  F  P  P  P  A
GGCGCCGCGATCCGGGCGGGGCCGCGGCGCCCAGGGTGACGGTGTCGGGCGGCAGCTTGTCCGCACCGCACGGCAATGTCATCGAGACCGGCGGCGGTGCGCGTCGCTTCCCGCCTCCGGCC
      1090      1100      1110      1120      1130      1140      1150      1160      1170      1180      1190      1200

  S  P  L  S  I  T  L  Q  A  G  A  R  A  Q  G  R  A  L  L  Y  R  V  L  P  E  P  V  K  L  T  L  A  G  G  A  Q  G  Q  G  Q
TCGCCCCTGTCGATCACCTTGCAGGCGGGCGCACGGGCGCAGGGGAGGGCGCTGCTGTACCGGGTCCTGCCGGAGCCCGTGAAGCTGACGCTGGCGGGCGGCGCCCAGGGGCAGGGCGAC
      1210      1220      1230      1240      1250      1260      1270      1280      1290      1300      1310      1320

  I  V  A  T  E  L  P  P  I  P  G  A  S  S  G  P  L  D  V  A  L  A  S  Q  A  R  W  T  G  A  T  R  A  V  D  S  L  S  I  D
ATCGTCGCGACGGAGCTGCCTCCCATTCCAGGCGCGTCGAGCGGGCCGCTCGACGTGGCGCTGGCCAGCCAGGCCCGATGGACGGGCGCTACCCGCGCGGTCGACTCGCTGTCCATCGAC
      1330      1340      1350     .1360      1370      1380      1390      1400      1410      1420      1430      1440

  N  A  T  W  V  M  T  D  N  S  N  V  G  A  L  R  L  A  S  D  G  S  V  D  F  Q  Q  P  A  E  A  G  R  F  K  V  L  M  V  D
AACGCCACCTGGGTCATGACGGACAACTCGAACGTCGGCGCGCTGCGGCTGGCCAGCGACGGCAGCGTCGATTTCCAGCAGCCGGCCGAAGCTGGGCGGTTCAAGGTCCTGATGGTCGAT
      1450      1460      1470      1480      1490      1500      1510      1520      1530      1540      1550      1560

  T  L  A  G  S  G  L  F  R  M  N  V  F  A  D  L  G  L  S  D  K  L  V  V  M  R  D  A  S  G  Q  M  R  L  W  V  R  N  S  G
ACGCTGGCGGGTTCGGGGCTGTTCCGCATGAATGTCTTCGCGGACCTGGGGCTGAGCGACAAGCTGGTCGTCATGCGGGACGCCAGCGGCCAGCACAGGCTGTGGGTCCGCAACAGCGGC
      1570      1580      1590      1600      1610      1620      1630      1640      1650      1660      1670      1680

  S  E  P  A  S  G  N  T  M  L  L  V  D  T  P  R  G  S  A  A  T  F  T  L  A  N  K  D  G  K  V  D  I  G  T  Y  R  Y  R  L
AGCGAGCCGGCCAGCGGCAACACCATGCTGCTGGTGCAGACGCCACGAGGCAGCGCGGCGACCTTTACCCTTGCCAACAAGGACGGCAAGGTCGATATCGGTACCTACCGCTATCGATTG
      1690      1700      1710      1720      1730      1740      1750      1760      1770      1780      1790      1800

  A  A  N  G  N  G  Q  W  S  L  V  G  A  K  A  P  P  A  P  K  P  A  P  Q  P  G  P  Q  P  G  P  Q  P  P  Q  P  P  Q  P  P
GCCGCCAACGGCAATGGGCAGTGGAGCCTGGTGGGCGCGAAGGCGCCGCCGGCGCCCAAGCCCGCGCCGCAGCCCGGTCCCCAGCCCGGTCCCCAGCCGCCGCAGCCGCCGCAGCCGCCG
      1810      1820      1830      1840      1850      1860      1870      1880      1890      1900      1910      1920

  Q  P  P  Q  P  P  Q  P  P  Q  R  Q  P  E  A  P  A  P  Q  P  P  P  A  G  R  E  L  S  A  A  A  N  A  A  V  N  T  G  V  G
CAGCCGCCGCAGCCGCCGCAGCCGCCCACAGAGGCAGCCGGAAGCGCCGGCGCCGCAACCGCCGGCGGGCAGGGAGTTGTCCGCCGCCGCCAACGCGGCGGTCAACACGGGTGGGGTGGGC
      1930      1940      1950      1960      1970      1980      1990      2000      2010      2020      2030      2040

  L  A  S  T  L  W  Y  A  E  S  N  A  L  S  K  R  L  G  E  L  R  L  N  P  D  A  G  G  A  W  G  R  G  F  A  Q  R  Q  Q  L
CTGGCCAGCACGCTCTGGTACGCCGAAAGCAATGCGTTGTCCAAGCGCCTGGGCGAGTTGCGCCTGAATCCGGACGCCGGCGGCGCTTGGGGCCGCGGCTTCGCGCAACGCCAGCAACTG
      2050      2060      2070      2080      2090       2100      2110      2120      2130      2140      2150      2160
```

# Fig.1 Cont (2).

```
     D  N  R  A  G  R  R  R  F  D  Q  K  V  A  G  F  E  L  G  A  D  H  A  V  A  V  A  G  G  R  W  H  L  G  G  L  A  G  Y  T  R
   GACAACCGCGCCGGGCGGCGCTTCGACCAGAAGGTGGCCGGCTTCGAGCTGGGCGCCGACCACGCGGTGGCGGTGGCCGGCGGGCGCTGGCACCTGGGCGGGGCTGGCCGGCTATACGCGC
       2170      2180      2190      2200      2210      2220      2230      2240      2250      2260      2270      2280


     G  D  R  G  F  T  G  D  G  G  G  H  T  D  S  V  H  V  G  G  Y  A  T  Y  I  A  N  S  G  F  V  L  D  A  T  L  R  A  S  R
   GGCGACCGCGGCTTTACCGGCGACGGCGGCGGCCACACCGACAGCGTGCATGTCGGGGGCTATGCCACCTATATCGCCAACAGCGGTTTCTACCTGGACGCGACGCTGCGCGCCAGCCGC
       2290      2300      2310      2320      2330      2340      2350      2360      2370      2380      2390      2400


     L .E  N  D  F  K  V  A  G  S  D  G  Y  A  V  K  G  K  Y  R  T  H  G  V  G  V  S  L  E  A  G  R  R  F  A  H  A  D  G  W
   CTCGAAAATGACTTCAAGGTGGCGGGCAGCGATGGGTACGCGGTCAAGGGCAAGTACCGCACCCATGGGGTAGGCGTCTCGCTCGAGGCGGGGCCGGCGCTTCGCCCATGCCGACGGCTGG
       2410      2420      2430      2440      2450      2460      2470      2480      2490      2500      2510      2520


     F  L  E  P  D  A  E  L  A  V  F  R  ,V  G  G  G  A  Y  R  A  A  N  G  L  R  V  R  D  E  G  G  S  S  V  L  G  R  L  G  L
   TTCCTCGAGCCGCAGGCCGAGCTGGCGGTGTTCCGGGTCGGCGGCGGTGCGTACCGCGCGGCCAATGGCCTGCGGGTGCGCGACGAAGGCGGCAGCTCGGTGCTGGGTCGCCTGGGCCTG
       2530      2540      2550      2560      2570      2580      2590      2600      2610      2620      2630      2640


     E  V  G  K  R  I  E  L  A  G  G  R  Q  V  Q  P  Y  V  I  K  A  S  V  L  Q  E  F  D  G  A  G  I  V  R  I  N  G  I  A  H  R
   GAGGTCGGCAAGCGCATCGAACTGGCAGGCGGCAGGCAGGTGCAGCCATACATCAAGGCCAGCGTGTTGCAGGAGTTCGACGGCGCGGGTACGGTACGCACCAACGGCATCGCGCATCGC
       2650      2660      2670      2680      2690      2700      2710      2720      2730      2740      2750      2760


     T  E  L  R  G  T  T  R  A  E  L  G  L  G  H  A  A  A  L  G  R  G  H  S  L  Y  A  S  V  E  Y  S  K  G  P  K  L  A  M  P  W
   ACCGAACTGCGCGGCACGCGCGCCGAACTGGGCCTGGGCATGGCCGCCGCGCTGGGCCGCGGCCACAGCCTGTATGCCTCGTACGAGTACTCCAAGGGCCCGAAGCTGGCCATGCCGTGG
       2770      2780      2790      2800      2810      2820      2830      2840      2850      2860      2870      2880


     T  F  H  A  G  V  R  Y  S  W  *
   ACCTTCCACGCGGGCTACCGGTACAGCTGGTAAAAGCGAGAAGGGTCCATCCCCCGCGGAGGAGTTTTTCCTGGAGGTTGGCCGGTGCCAGTCTCCAGGCTCAGGCGGCCAGGGCCTGCGG
       2890      2900      2910      2920      2930      2940      2950      2960      2970      2980      2990      3000
```

EP 0 789 031 A2

# Fig. 2.

0kb     1kb     2kb     3kb

ClaI   AlfIII   SmaI   PstI   SalI   ClaI   BglI   BssHII   BglI   BssHII   BglI

CLONED

OLIGO PRIMED

ORF of p.95

EP 0 789 031 A2

# Fig.3.

SEQUENCE LISTING


(1) GENERAL INFORMATION:

    (i) APPLICANT:
        (A) NAME: Evans Medical Limited
        (B) STREET: Evans House, Regent Park, Kingston Road
        (C) CITY: Leatherhead
        (D) STATE: Surrey
        (E) COUNTRY: United kingdom
        (F) POSTAL CODE (ZIP): KT22 7PQ


    (ii) TITLE OF INVENTION: ACELLULAR VACCINE


    (iii) NUMBER OF SEQUENCES: 2


    (iv) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.30 (EPO)


(2) INFORMATION FOR SEQ ID NO: 1:

    (i) SEQUENCE CHARACTERISTICS:
        (A) LENGTH: 3000 base pairs
        (B) TYPE: nucleic acid
        (C) STRANDEDNESS: double
        (D) TOPOLOGY: linear

    (ii) MOLECULE TYPE: DNA (genomic)


    (ix) FEATURE:
        (A) NAME/KEY: CDS
        (B) LOCATION:145..2910


    (xi) SEQUENCE DESCRIPTION: SEQ ID NO: 1:

ATCGATGATG CGTCGCTGTA ACACGGCAAA TACCGTGCAT TGCAGCGGTT CTGGATGGCG      60

TTCTTCGTAC GTTTGCTGCG CCCATTCTTC CCTGTTCCAT CGCGGTGCGG GCATGGCGGG     120

```
CGTCTGCTCT TCACCCGGCA TCCA ATG AAC ATG TCT CTG TCA CGC ATT GTC          171
                         Met Asn Met Ser Leu Ser Arg Ile Val
                          1                   5

AAG GCG GCG CCC CTG CGC CGC ACC ACA CTG GCC ATG GCG CTG GGC GCG          219
Lys Ala Ala Pro Leu Arg Arg Thr Thr Leu Ala Met Ala Leu Gly Ala
 10                 15                  20                  25

CTG GGC GCC GCG CCC GCC GCG TAC GCC GAC TGG AAC AAC CAG TCC ATC          267
Leu Gly Ala Ala Pro Ala Ala Tyr Ala Asp Trp Asn Asn Gln Ser Ile
                30                  35                  40

ATC AAG GCC GGC GAG CGC CAG CAC GGC ATC CAC ATC AAG CAA AGC GAT          315
Ile Lys Ala Gly Glu Arg Gln His Gly Ile His Ile Lys Gln Ser Asp
                45                  50                  55

GGC GCC GGC GTA CGG ACC GCC ACC GGA ACG ACC ATC AAG GTA AGC GGT          363
Gly Ala Gly Val Arg Thr Ala Thr Gly Thr Thr Ile Lys Val Ser Gly
            60                  65                  70

CGT CAG GCC CAG GGC GTC CTG CTG GAA AAT CCC GCG GCC GAG CTG CGG          411
Arg Gln Ala Gln Gly Val Leu Leu Glu Asn Pro Ala Ala Glu Leu Arg
        75                  80                  85

TTC CAG AAC GGC AGC GTC ACG TCT TCG GGA CAG CTG TTC GAC GAA GGC          459
Phe Gln Asn Gly Ser Val Thr Ser Ser Gly Gln Leu Phe Asp Glu Gly
 90                 95                  100                 105

GTC CGG CGC TTT CTG GGC ACC GTC ACC GTC AAG GCC GGC AAG CTG GTC          507
Val Arg Arg Phe Leu Gly Thr Val Thr Val Lys Ala Gly Lys Leu Val
                110                 115                 120

GCC GAT CAC GCC ACG CTG GCC AAC GTC AGC GAC ACC CGG GAC GAC GAC          555
Ala Asp His Ala Thr Leu Ala Asn Val Ser Asp Thr Arg Asp Asp Asp
                125                 130                 135

GGC ATC GCG CTC TAT GTG GCC GGC GAG CAG GCC CAG GCC AGC ATC GCC          603
Gly Ile Ala Leu Tyr Val Ala Gly Glu Gln Ala Gln Ala Ser Ile Ala
            140                 145                 150

GAC AGC ACC CTG CAG GGC GCG GGC GGC GTG CGG GTC GAG CGC GGC GCC          651
Asp Ser Thr Leu Gln Gly Ala Gly Gly Val Arg Val Glu Arg Gly Ala
        155                 160                 165

AAT GTC ACG GTC CAA CGC AGC ACC ATC GTT GAC GGG GGC TTG CAT ATC          699
Asn Val Thr Val Gln Arg Ser Thr Ile Val Asp Gly Gly Leu His Ile
170                 175                 180                 185
```

```
GGC ACC CTG CAG CCG CTG CAG CCG GAA GAC CTT CCG CCC AGC CGG GTG          747
Gly Thr Leu Gln Pro Leu Gln Pro Glu Asp Leu Pro Pro Ser Arg Val
            190                 195                 200

GTG CTG GGC GAC ACC AGC GTG ACC GCC GTG CCC GCC AGC GGC GCG CCC          795
Val Leu Gly Asp Thr Ser Val Thr Ala Val Pro Ala Ser Gly Ala Pro
            205                 210                 215

GCG GCG GTG TTT GTA TTC GGG GCC AAT GAG CTT ACG GTT GAT GGC GGG          843
Ala Ala Val Phe Val Phe Gly Ala Asn Glu Leu Thr Val Asp Gly Gly
        220                 225                 230

CAC ATC ACC GGG GGG CGG GCA GCG GGG GTG GCG GCC ATG GAC GGG GCG          891
His Ile Thr Gly Gly Arg Ala Ala Gly Val Ala Ala Met Asp Gly Ala
        235                 240                 245

ATC GTG CAT CTG CAG CGC GCG ACG ATA CGG CGG GGG GAC GCG CCT GCC          939
Ile Val His Leu Gln Arg Ala Thr Ile Arg Arg Gly Asp Ala Pro Ala
250                 255                 260                 265

GGC GGT GCG GTT CCA GGC GGT GCG GTT CCC GGC GGT GCC GTT CCC GGC          987
Gly Gly Ala Val Pro Gly Gly Ala Val Pro Gly Gly Ala Val Pro Gly
            270                 275                 280

GGC TTC GGC CCC CTC CTT GAC GGC TGG TAT GGC GTG GAT GTA TCG GAC         1035
Gly Phe Gly Pro Leu Leu Asp Gly Trp Tyr Gly Val Asp Val Ser Asp
            285                 290                 295

TCC ACC GTG GAC CTC GCT CAG TCG ATC GTC GAG GCG CCG CAG CTG GGC         1083
Ser Thr Val Asp Leu Ala Gln Ser Ile Val Glu Ala Pro Gln Leu Gly
            300                 305                 310

GCC GCG ATC CGG GCG GGC CGC GGC GCC AGG GTG ACG GTG TCG GGC GGC         1131
Ala Ala Ile Arg Ala Gly Arg Gly Ala Arg Val Thr Val Ser Gly Gly
        315                 320                 325

AGC TTG TCC GCA CCG CAC GGC AAT GTC ATC GAG ACC GGC GGC GGT GCG         1179
Ser Leu Ser Ala Pro His Gly Asn Val Ile Glu Thr Gly Gly Gly Ala
330                 335                 340                 345

CGT CGC TTC CCG CCT CCG GCC TCG CCC CTG TCG ATC ACC TTG CAG GCG         1227
Arg Arg Phe Pro Pro Pro Ala Ser Pro Leu Ser Ile Thr Leu Gln Ala
            350                 355                 360

GGC GCA CGG GCG CAG GGG AGG GCG CTG CTG TAC CGG GTC CTG CCG GAG         1275
Gly Ala Arg Ala Gln Gly Arg Ala Leu Leu Tyr Arg Val Leu Pro Glu
            365                 370                 375
```

```
CCC GTG AAG CTG ACG CTG GCG GGC GGC GCC CAG GGG CAG GGC GAC ATC          1323
Pro Val Lys Leu Thr Leu Ala Gly Gly Ala Gln Gly Gln Gly Asp Ile
        380             385             390

GTC GCG ACG GAG CTG CCT CCC ATT CCA GGC GCG TCG AGC GGG CCG CTC          1371
Val Ala Thr Glu Leu Pro Pro Ile Pro Gly Ala Ser Ser Gly Pro Leu
        395             400             405

GAC GTG GCG CTG GCC AGC CAG GCC CGA TGG ACG GGC GCT ACC CGC GCG          1419
Asp Val Ala Leu Ala Ser Gln Ala Arg Trp Thr Gly Ala Thr Arg Ala
410             415             420             425

GTC GAC TCG CTG TCC ATC GAC AAC GCC ACC TGG GTC ATG ACG GAC AAC          1467
Val Asp Ser Leu Ser Ile Asp Asn Ala Thr Trp Val Met Thr Asp Asn
            430             435             440

TCG AAC GTC GGC GCG CTG CGG CTG GCC AGC GAC GGC AGC GTC GAT TTC          1515
Ser Asn Val Gly Ala Leu Arg Leu Ala Ser Asp Gly Ser Val Asp Phe
            445             450             455

CAG CAG CCG GCC GAA GCT GGG CGG TTC AAG GTC CTG ATG GTC GAT ACG          1563
Gln Gln Pro Ala Glu Ala Gly Arg Phe Lys Val Leu Met Val Asp Thr
        460             465             470

CTG GCG GGT TCG GGG CTG TTC CGC ATG AAT GTC TTC GCG GAC CTG GGG          1611
Leu Ala Gly Ser Gly Leu Phe Arg Met Asn Val Phe Ala Asp Leu Gly
        475             480             485

CTG AGC GAC AAG CTG GTC GTC ATG CGG GAC GCC AGC GGC CAG CAC AGG          1659
Leu Ser Asp Lys Leu Val Val Met Arg Asp Ala Ser Gly Gln His Arg
490             495             500             505

CTG TGG GTC CGC AAC AGC GGC AGC GAG CCG GCC AGC GGC AAC ACC ATG          1707
Leu Trp Val Arg Asn Ser Gly Ser Glu Pro Ala Ser Gly Asn Thr Met
            510             515             520

CTG CTG GTG CAG ACG CCA CGA GGC AGC GCG GCG ACC TTT ACC CTT GCC          1755
Leu Leu Val Gln Thr Pro Arg Gly Ser Ala Ala Thr Phe Thr Leu Ala
            525             530             535

AAC AAG GAC GGC AAG GTC GAT ATC GGT ACC TAC CGC TAT CGA TTG GCC          1803
Asn Lys Asp Gly Lys Val Asp Ile Gly Thr Tyr Arg Tyr Arg Leu Ala
            540             545             550

GCC AAC GGC AAT GGG CAG TGG AGC CTG GTG GGC GCG AAG GCG CCG CCG          1851
Ala Asn Gly Asn Gly Gln Trp Ser Leu Val Gly Ala Lys Ala Pro Pro
        555             560             565
```

```
GCG CCC AAG CCC GCG CCG CAG CCC GGT CCC CAG CCC GGT CCC CAG CCG          1899
Ala Pro Lys Pro Ala Pro Gln Pro Gly Pro Gln Pro Gly Pro Gln Pro
570             575             580             585

CCG CAG CCG CCG CAG CCG CCG CAG CCG CCG CAG CCG CCG CAG CCG CCA          1947
Pro Gln Pro Pro Gln Pro Pro Gln Pro Pro Gln Pro Pro Gln Pro Pro
                590             595             600

CAG AGG CAG CCG GAA GCG CCG GCG CCG CAA CCG CCG GCG GGC AGG GAG          1995
Gln Arg Gln Pro Glu Ala Pro Ala Pro Gln Pro Pro Ala Gly Arg Glu
            605             610             615

TTG TCC GCC GCC GCC AAC GCG GCG GTC AAC ACG GGT GGG GTG GGC CTG          2043
Leu Ser Ala Ala Ala Asn Ala Ala Val Asn Thr Gly Gly Val Gly Leu
        620             625             630

GCC AGC ACG CTC TGG TAC GCC GAA AGC AAT GCG TTG TCC AAG CGC CTG          2091
Ala Ser Thr Leu Trp Tyr Ala Glu Ser Asn Ala Leu Ser Lys Arg Leu
        635             640             645

GGC GAG TTG CGC CTG AAT CCG GAC GCC GGC GGC GCT TGG GGC CGC GGC          2139
Gly Glu Leu Arg Leu Asn Pro Asp Ala Gly Gly Ala Trp Gly Arg Gly
650             655             660             665

TTC GCG CAA CGC CAG CAA CTG GAC AAC CGC GCC GGG CGG CGC TTC GAC          2187
Phe Ala Gln Arg Gln Gln Leu Asp Asn Arg Ala Gly Arg Arg Phe Asp
                670             675             680

CAG AAG GTG GCC GGC TTC GAG CTG GGC GCC GAC CAC GCG GTG GCG GTG          2235
Gln Lys Val Ala Gly Phe Glu Leu Gly Ala Asp His Ala Val Ala Val
            685             690             695

GCC GGC GGG CGC TGG CAC CTG GGC GGG CTG GCC GGC TAT ACG CGC GGC          2283
Ala Gly Gly Arg Trp His Leu Gly Gly Leu Ala Gly Tyr Thr Arg Gly
        700             705             710

GAC CGC GGC TTT ACC GGC GAC GGC GGC GGC CAC ACC GAC AGC GTG CAT          2331
Asp Arg Gly Phe Thr Gly Asp Gly Gly Gly His Thr Asp Ser Val His
        715             720             725

GTC GGG GGC TAT GCC ACC TAT ATC GCC AAC AGC GGT TTC TAC CTG GAC          2379
Val Gly Gly Tyr Ala Thr Tyr Ile Ala Asn Ser Gly Phe Tyr Leu Asp
730             735             740             745

GCG ACG CTG CGC GCC AGC CGC CTC GAA AAT GAC TTC AAG GTG GCG GGC          2427
Ala Thr Leu Arg Ala Ser Arg Leu Glu Asn Asp Phe Lys Val Ala Gly
                750             755             760
```

```
AGC GAT GGG TAC GCG GTC AAG GGC AAG TAC CGC ACC CAT GGG GTA GGC        2475
Ser Asp Gly Tyr Ala Val Lys Gly Lys Tyr Arg Thr His Gly Val Gly
            765                 770                 775

GTC TCG CTC GAG GCG GGC CGG CGC TTC GCC CAT GCC GAC GGC TGG TTC        2523
Val Ser Leu Glu Ala Gly Arg Arg Phe Ala His Ala Asp Gly Trp Phe
            780                 785                 790

CTC GAG CCG CAG GCC GAG CTG GCG GTG TTC CGG GTC GGC GGC GGT GCG        2571
Leu Glu Pro Gln Ala Glu Leu Ala Val Phe Arg Val Gly Gly Gly Ala
    795                 800                 805

TAC CGC GCG GCC AAT GGC CTG CGG GTG CGC GAC GAA GGC GGC AGC TCG        2619
Tyr Arg Ala Ala Asn Gly Leu Arg Val Arg Asp Glu Gly Gly Ser Ser
810                 815                 820                 825

GTG CTG GGT CGC CTG GGC CTG GAG GTC GGC AAG CGC ATC GAA CTG GCA        2667
Val Leu Gly Arg Leu Gly Leu Glu Val Gly Lys Arg Ile Glu Leu Ala
                830                 835                 840

GGC GGC AGG CAG GTG CAG CCA TAC ATC AAG GCC AGC GTG TTG CAG GAG        2715
Gly Gly Arg Gln Val Gln Pro Tyr Ile Lys Ala Ser Val Leu Gln Glu
            845                 850                 855

TTC GAC GGC GCG GGT ACG GTA CGC ACC AAC GGC ATC GCG CAT CGC ACC        2763
Phe Asp Gly Ala Gly Thr Val Arg Thr Asn Gly Ile Ala His Arg Thr
            860                 865                 870

GAA CTG CGC GGC ACG CGC GCC GAA CTG GGC CTG GGC ATG GCC GCC GCG        2811
Glu Leu Arg Gly Thr Arg Ala Glu Leu Gly Leu Gly Met Ala Ala Ala
    875                 880                 885

CTG GGC CGC GGC CAC AGC CTG TAT GCC TCG TAC GAG TAC TCC AAG GGC        2859
Leu Gly Arg Gly His Ser Leu Tyr Ala Ser Tyr Glu Tyr Ser Lys Gly
890                 895                 900                 905

CCG AAG CTG GCC ATG CCG TGG ACC TTC CAC GCG GGC TAC CGG TAC AGC        2907
Pro Lys Leu Ala Met Pro Trp Thr Phe His Ala Gly Tyr Arg Tyr Ser
                910                 915                 920

TGG TAAAGCGAGA AGGGTCCATC CCCCGCGGAG GAGTTTTTCC TGGAGGTTGG            2960
Trp


CCGGTGCCAG TCTCCAGGCT CAGGCGGCCA GGGCCTGCGG                           3000
```

(2) INFORMATION FOR SEQ ID NO: 2:

    (i) SEQUENCE CHARACTERISTICS:
        (A) LENGTH: 922 amino acids
        (B) TYPE: amino acid
        (D) TOPOLOGY: linear

    (ii) MOLECULE TYPE: protein
    (xi) SEQUENCE DESCRIPTION: SEQ ID NO: 2:

Met Asn Met Ser Leu Ser Arg Ile Val Lys Ala Ala Pro Leu Arg Arg
 1               5                  10                  15

Thr Thr Leu Ala Met Ala Leu Gly Ala Leu Gly Ala Ala Pro Ala Ala
            20                  25                  30

Tyr Ala Asp Trp Asn Asn Gln Ser Ile Ile Lys Ala Gly Glu Arg Gln
        35                  40                  45

His Gly Ile His Ile Lys Gln Ser Asp Gly Ala Gly Val Arg Thr Ala
        50                  55                  60

Thr Gly Thr Thr Ile Lys Val Ser Gly Arg Gln Ala Gln Gly Val Leu
 65                  70                  75                  80

Leu Glu Asn Pro Ala Ala Glu Leu Arg Phe Gln Asn Gly Ser Val Thr
            85                  90                  95

Ser Ser Gly Gln Leu Phe Asp Glu Gly Val Arg Arg Phe Leu Gly Thr
            100                 105                 110

Val Thr Val Lys Ala Gly Lys Leu Val Ala Asp His Ala Thr Leu Ala
            115                 120                 125

Asn Val Ser Asp Thr Arg Asp Asp Gly Ile Ala Leu Tyr Val Ala
        130                 135                 140

Gly Glu Gln Ala Gln Ala Ser Ile Ala Asp Ser Thr Leu Gln Gly Ala
145                 150                 155                 160

Gly Gly Val Arg Val Glu Arg Gly Ala Asn Val Thr Val Gln Arg Ser
            165                 170                 175

Thr Ile Val Asp Gly Gly Leu His Ile Gly Thr Leu Gln Pro Leu Gln
            180                 185                 190

Pro Glu Asp Leu Pro Pro Ser Arg Val Val Leu Gly Asp Thr Ser Val
        195                 200                 205

Thr Ala Val Pro Ala Ser Gly Ala Pro Ala Ala Val Phe Val Phe Gly

```
                210                    215                    220

Ala Asn Glu Leu Thr Val Asp Gly Gly His Ile Thr Gly Gly Arg Ala
225                 230                 235                    240

Ala Gly Val Ala Ala Met Asp Gly Ala Ile Val His Leu Gln Arg Ala
                245                 250                    255

Thr Ile Arg Arg Gly Asp Ala Pro Ala Gly Gly Ala Val Pro Gly Gly
            260                 265                 270

Ala Val Pro Gly Gly Ala Val Pro Gly Gly Phe Gly Pro Leu Leu Asp
            275                 280                 285

Gly Trp Tyr Gly Val Asp Val Ser Asp Ser Thr Val Asp Leu Ala Gln
    290                 295                 300

Ser Ile Val Glu Ala Pro Gln Leu Gly Ala Ala Ile Arg Ala Gly Arg
305                 310                 315                    320

Gly Ala Arg Val Thr Val Ser Gly Gly Ser Leu Ser Ala Pro His Gly
                325                 330                    335

Asn Val Ile Glu Thr Gly Gly Gly Ala Arg Arg Phe Pro Pro Pro Ala
            340                 345                 350

Ser Pro Leu Ser Ile Thr Leu Gln Ala Gly Ala Arg Ala Gln Gly Arg
            355                 360                 365

Ala Leu Leu Tyr Arg Val Leu Pro Glu Pro Val Lys Leu Thr Leu Ala
    370                 375                 380

Gly Gly Ala Gln Gly Gln Gly Asp Ile Val Ala Thr Glu Leu Pro Pro
385                 390                 395                    400

Ile Pro Gly Ala Ser Ser Gly Pro Leu Asp Val Ala Leu Ala Ser Gln
                405                 410                    415

Ala Arg Trp Thr Gly Ala Thr Arg Ala Val Asp Ser Leu Ser Ile Asp
            420                 425                 430

Asn Ala Thr Trp Val Met Thr Asp Asn Ser Asn Val Gly Ala Leu Arg
        435                 440                 445

Leu Ala Ser Asp Gly Ser Val Asp Phe Gln Gln Pro Ala Glu Ala Gly
    450                 455                 460

Arg Phe Lys Val Leu Met Val Asp Thr Leu Ala Gly Ser Gly Leu Phe
```

```
        465                   470                   475                   480

Arg Met Asn Val Phe Ala Asp Leu Gly Leu Ser Asp Lys Leu Val Val
                485                   490                   495

Met Arg Asp Ala Ser Gly Gln His Arg Leu Trp Val Arg Asn Ser Gly
                500                   505                   510

Ser Glu Pro Ala Ser Gly Asn Thr Met Leu Leu Val Gln Thr Pro Arg
                515                   520                   525

Gly Ser Ala Ala Thr Phe Thr Leu Ala Asn Lys Asp Gly Lys Val Asp
        530                   535                   540

Ile Gly Thr Tyr Arg Tyr Arg Leu Ala Ala Asn Gly Asn Gly Gln Trp
545                   550                   555                   560

Ser Leu Val Gly Ala Lys Ala Pro Pro Ala Pro Lys Pro Ala Pro Gln
                565                   570                   575

Pro Gly Pro Gln Pro Gly Pro Gln Pro Pro Gln Pro Pro Gln Pro Pro
                580                   585                   590

Gln Pro Pro Gln Pro Pro Gln Pro Pro Gln Arg Gln Pro Glu Ala Pro
        595                   600                   605

Ala Pro Gln Pro Pro Ala Gly Arg Glu Leu Ser Ala Ala Ala Asn Ala
        610                   615                   620

Ala Val Asn Thr Gly Gly Val Gly Leu Ala Ser Thr Leu Trp Tyr Ala
625                   630                   635                   640

Glu Ser Asn Ala Leu Ser Lys Arg Leu Gly Glu Leu Arg Leu Asn Pro
                645                   650                   655

Asp Ala Gly Gly Ala Trp Gly Arg Gly Phe Ala Gln Arg Gln Gln Leu
                660                   665                   670

Asp Asn Arg Ala Gly Arg Arg Phe Asp Gln Lys Val Ala Gly Phe Glu
                675                   680                   685

Leu Gly Ala Asp His Ala Val Ala Val Ala Gly Gly Arg Trp His Leu
        690                   695                   700

Gly Gly Leu Ala Gly Tyr Thr Arg Gly Asp Arg Gly Phe Thr Gly Asp
705                   710                   715                   720

Gly Gly Gly His Thr Asp Ser Val His Val Gly Gly Tyr Ala Thr Tyr
```

```
                725                      730                      735

   Ile Ala Asn Ser Gly Phe Tyr Leu Asp Ala Thr Leu Arg Ala Ser Arg
               740                      745                 750

   Leu Glu Asn Asp Phe Lys Val Ala Gly Ser Asp Gly Tyr Ala Val Lys
               755                      760                 765

   Gly Lys Tyr Arg Thr His Gly Val Gly Val Ser Leu Glu Ala Gly Arg
           770                      775                 780

   Arg Phe Ala His Ala Asp Gly Trp Phe Leu Glu Pro Gln Ala Glu Leu
   785                      790                 795                 800

   Ala Val Phe Arg Val Gly Gly Gly Ala Tyr Arg Ala Ala Asn Gly Leu
                   805                      810                 815

   Arg Val Arg Asp Glu Gly Gly Ser Ser Val Leu Gly Arg Leu Gly Leu
                   820                      825                 830

   Glu Val Gly Lys Arg Ile Glu Leu Ala Gly Gly Arg Gln Val Gln Pro
               835                      840                 845

   Tyr Ile Lys Ala Ser Val Leu Gln Glu Phe Asp Gly Ala Gly Thr Val
           850                      855                 860

   Arg Thr Asn Gly Ile Ala His Arg Thr Glu Leu Arg Gly Thr Arg Ala
   865                      870                 875                 880

   Glu Leu Gly Leu Gly Met Ala Ala Ala Leu Gly Arg Gly His Ser Leu
                   885                      890                 895

   Tyr Ala Ser Tyr Glu Tyr Ser Lys Gly Pro Lys Leu Ala Met Pro Trp
               900                      905                 910

   Thr Phe His Ala Gly Tyr Arg Tyr Ser Trp
           915                      920
```